# EUROPEAN PATENT APPLICATION

(11) **EP 4 349 354 A1**
(43) Date of publication of application: **10.04.2024**
(21) Application number: 22816454.7
(22) Date of filing: 31.05.2022
(51) Int. Cl.: A61K 35/747, A61K 9/00, A61P 37/00, A61P 3/00, A23L 33/135, A61K 8/99, A61Q 19/00

(54) **EXTRACELLULAR VESICLES DERIVED FROM LACTOBACILLUS BACTERIA, AND USE THEREOF**

(30) Priority: 03.06.2021 KR 20210072078; 23.12.2021 KR 20210186256
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2022/007786
(87) International publication number: WO 2022/255795

(57) **Abstract**

The present invention relates to a composition for preventing or treating diseases caused by immune dysfunction and metabolic dysfunction, including *Lactobacillus* bacteria-derived extracellular vesicles as an active ingredient, and the *Lactobacillus* bacteria-derived vesicles according to the present invention may be distributed to major organs after being absorbed into the human body, and after being absorbed into cells, they may move to organelles to inhibit NF-xB signaling, which is a key signal of inflammatory responses, thereby regulating inflammatory responses. In addition, since the vesicles according to the present invention increase cell viability by activating AMPK signaling in metabolic stress situations where ATP production is poor, it is expected that the vesicles may be effectively used in the development of pharmaceuticals or health functional foods for alleviating, preventing, or treating diseases caused by immune dysfunction or metabolic dysfunction.

## Description

### [Technical Field]

The present invention relates to extracellular vesicles derived from *Lactobacillus* bacteria and a use thereof, and more specifically, to a composition for preventing or treating immune or metabolic dysfunction-related diseases, comprising extracellular vesicles derived from *Lactobacillus* bacteria as an active ingredient.

This application claims priority to and the benefit of Korean Patent Application Nos. 10-2021-0072078 and 10-2021-0186256 filed in the Korean Intellectual Property Office on June 3, 2021 and December 23, 2021, respectively, and all the contents disclosed in the specification and drawings of the applications are incorporated in this application.

### [Background Art]

Entering the twenty-first century, the importance of acute infectious diseases, which were recognized as contagious diseases in the past, has decreased. On the other hand, the disease pattern has changed in that chronic diseases caused by immune dysfunction and cellular aging in major organs of our body have become major diseases that reduce the quality of life and determine the human lifespan. Intractable chronic diseases that occur in major organs such as the skin, gastrointestinal tract, heart, lungs, liver, kidneys, musculoskeletal system, and brain are characterized by inflammation and cell death accompanied by immune dysfunction and metabolic dysfunction.

Immunity, which is a cellular defense mechanism against biological, chemical, physical, and mental stresses, occurs through innate immunity and adaptive immunity. Metabolism is production of various substances that perform cellular functions by generating the energy needed for our body, wherein proteins and lipids are produced in the endoplasmic reticulum (ER) through ATP produced in the mitochondria, thereby supplying substances to areas where they are needed. Cells face various stresses from the moment they are formed. Biological, chemical, physical, and mental stresses induce ER stress within cells, and when ER stress persists, cells die or turn into cancer cells. Therefore, it has recently been revealed that many intractable diseases that are problematic are caused by immune dysfunction and metabolic dysfunction due to repetitive ER stress.

Meanwhile, "lactic acid bacteria" collectively refers to bacteria that decompose carbohydrates into lactic acid through metabolism, and lactic acid bacteria ferment foods such as yogurt, lactic acid bacteria drinks, and kimchi. It is known that some lactic acid bacteria are present in digestive organs such as the intestines (enteric bacteria) or in the vagina, where they protect the body from pathogenic microorganisms and help maintain homeostasis. Among these, *Lactobacillus* bacteria are aerobic bacteria that do not form spores, and they are known to live symbiotically in the oral cavity, gastrointestinal tract, and female vagina and play a role in protecting our body from pathogenic bacteria.

When cells fail to properly defend themselves against various stresses, inflammation occurs due to immune dysfunction, and chronic inflammatory diseases occur due to repetitive stress. Compositions that are generally used to treat or prevent chronic inflammatory diseases are largely divided into steroidal and non-steroidal compositions, and in particular, steroid preparations often have various adverse effects when used for a long period of time. Therefore, there is a recent trend of increasing interest in TNF-α or IL-6 inhibitors or the like, which are inflammatory cytokines known to be major mediators of chronic inflammatory diseases. However, the development of drugs that can be effectively used to prevent and treat chronic inflammatory diseases by effectively inhibiting the expression of TNF-α and IL-6 is still insufficient.

### [Disclosure]

### [Technical Problem]

The present invention has been developed to solve the problems of the related art as described above, and an object of the present invention is to provide a composition for alleviating, preventing or treating immune or metabolic dysfunction-related diseases, comprising extracellular vesicles (EVs), which are *Lactobacillus* bacteria-derived materials, as an active ingredient.

However, a technical problem to be achieved by the present invention is not limited to the aforementioned problems, and the other problems that are not mentioned may be clearly understood by a person skilled in the art from the following description.

### [Technical Solution]

The present invention is to provide a pharmaceutical composition for preventing or treating immune or metabolic dysfunction-related diseases, comprising vesicles derived from *Lactobacillus* bacteria as an active ingredient.

In addition, the present invention is to provide a food composition for preventing or alleviating immune or metabolic dysfunction-related diseases; or aging, comprising vesicles derived from *Lactobacillus* bacteria as an active ingredient.

In addition, the present invention is to provide an inhalant composition for preventing or alleviating immune or metabolic dysfunction-related diseases, comprising vesicles derived from *Lactobacillus* bacteria as an active ingredient.

In addition, the present invention is to provide a cosmetic composition for preventing or alleviating immune or metabolic dysfunction-related diseases; or skin aging, comprising vesicles derived from *Lactobacillus* bacteria as an active ingredient.

In one embodiment of the present invention, the *Lactobacillus* bacteria may be one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae,* but are not limited thereto.

In another embodiment of the present invention, the immune or metabolic dysfunction-related disease may be one or more selected from the group consisting of immune diseases, metabolic diseases, and malignant diseases, but is not limited to thereto.

In still another embodiment of the present invention, the immune or metabolic dysfunction-related disease may be selected from the group consisting of the following diseases, but is not limited thereto:
one or more skin diseases selected from the group consisting of atopic dermatitis, psoriasis, acne, and alopecia; one or more oral diseases selected from the group consisting of gingivitis and periodontitis; one or more gastrointestinal diseases selected from the group consisting of gastritis, peptic ulcers, celiac disease, food allergies, and inflammatory bowel disease; one or more hepato-biliary-pancreatic diseases selected from the group consisting of non-alcoholic steatohepatitis (NASH), alcoholic steatohepatitis, liver cirrhosis, cholangitis, cholecystitis, and pancreatitis; one or more nasal diseases selected from the group consisting of rhinitis and sinusitis; one or more pulmonary diseases selected from the group consisting of asthma, chronic obstructive pulmonary disease (COPD), pneumonia, chronic interstitial pneumonitis, and pulmonary fibrosis; one or more cardiovascular diseases selected from the group consisting of atherosclerosis, angina, metabolic syndrome, thromboembolism, myocardial infarction, cardiomyopathy, and stroke; one or more musculoskeletal diseases selected from the group consisting of rheumatoid arthritis, osteoarthritis, gout, sarcopenia, and osteoporosis; one or more neuro-psychiatric disorders selected from the group consisting of Alzheimer's disease, vascular dementia, Parkinson's disease, amyotrophic lateral sclerosis, Huntington's disease, epilepsy, multiple sclerosis, chronic inflammatory demyelinating polyneuropathy, diabetic neuropathy, autism spectrum disorder, attention deficit hyperactivity syndrome, depressive disorder, bipolar disorder, anxiety disorders, schizophrenia, obsessive compulsive disorder, post-traumatic stress disorder, dissociative disorders, eating disorders, substance use disorder, and personality disorders; and one or more ocular diseases selected from the group consisting of macular degeneration, diabetic retinopathy, glaucoma, cataracts, and dry eye.

In yet another embodiment of the present invention, the immune disease may be one or more selected from the group consisting of one or more infectious diseases selected from the group consisting of viral infections and bacterial infections; one or more allergic diseases selected from the group consisting of atopic dermatitis, allergic rhinitis, asthma, hypersensitivity pneumonitis, food allergies, and anaphylaxis; and one or more autoimmune diseases selected from the group consisting of systemic lupus erythematosus, rheumatoid arthritis, multiple sclerosis, and Behcet's disease, but is not limited thereto.

In yet another embodiment of the present invention, the metabolic disease may be one or more selected from the group consisting of diabetes, metabolic syndrome, lipid metabolism abnormalities, arteriosclerosis, and obesity, but is not limited thereto.

In yet another embodiment of the present invention, the malignant disease may be one or more selected from the group consisting of lung cancer, head and neck cancer, esophageal cancer, stomach cancer, colorectal cancer, colon cancer, liver cancer, bile duct cancer, pancreatic cancer, breast cancer, ovarian cancer, kidney cancer, bladder cancer, prostate cancer, thyroid cancer, brain tumors, osteosarcoma, hematologic cancer, and lymphoma, but is not limited thereto.

In yet another embodiment of the present invention, the immune or metabolic dysfunction-related disease may be a disease mediated by inducible NO synthase (iNOS), but is not limited thereto.

In yet another embodiment of the present invention, the immune or metabolic dysfunction-related disease may be a disease mediated by TNF-α or IL-6, but is not limited thereto.

As yet another exemplary embodiment of the present invention, the vesicles may have an average diameter of 10 to 300 nm, but are not limited thereto.

As another exemplary embodiment of the present invention, the vesicles may be naturally secreted or artificially produced from *Lactobacillus* bacteria, but the present invention is not limited thereto.

In yet another embodiment of the present invention, the vesicles may be isolated from a culture medium of *Lactobacillus* bacteria or food cultured by adding *Lactobacillus* bacteria, but are not limited thereto.

In addition, the present invention is provides a composition for delivering a disease-treating drug, comprising vesicles derived from *Lactobacillus* bacteria as an active ingredient.

In addition, the present invention provides a method for preventing or treating immune or metabolic dysfunction-related diseases, the method comprising administering a composition comprising vesicles derived from *Lactobacillus* bacteria as an active ingredient to a subject in need thereof.

In addition, the present invention provides a use of a composition comprising vesicles derived from *Lactobacillus* bacteria as an active ingredient for preventing or treating immune or metabolic dysfunction-related diseases.

In addition, the present invention provides a use of vesicles derived from *Lactobacillus* bacteria for producing drugs for treating immune or metabolic dysfunction-related diseases.

In addition, the present invention provides a disease-treating drug delivery system, comprising *Lactobacillus* bacteria-derived vesicles as an active ingredient.

In addition, the present invention provides a method of delivering a drug for treating disease, which comprises administering a composition comprising *Lactobacillus* bacteria-derived vesicles carrying a desired disease-treating drug as an active ingredient to a subject in need thereof.

In addition, the present invention provides a use of a composition comprising *Lactobacillus* bacteria-derived vesicles as an active ingredient for delivering a diseasetreating drug.

In addition, the present invention provides a use of *Lactobacillus* bacteria-derived vesicles for preparing a preparation for delivering a disease-treating drug.

### [Advantageous Effects]

*Lactobacillus* bacteria-derived vesicles according to the present invention can be distributed to major organs after being absorbed into the human body, and after being absorbed into cells, they can move to organelles to inhibit NF-κB signaling, which is a key signal of inflammatory responses, thereby regulating inflammatory responses. In addition, since the vesicles according to the present invention increase cellular homeostasis by activating AMPK signaling in metabolic stress situations where ATP production is poor, it is expected that the vesicles may be effectively used in the development of pharmaceuticals or health functional foods for alleviating, preventing, or treating immune or metabolic dysfunction-related diseases.

### [Description of Drawings]

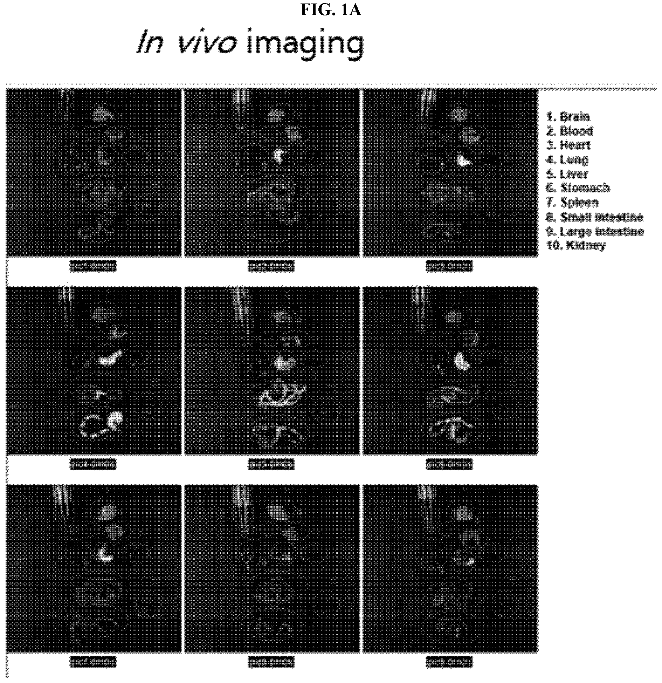

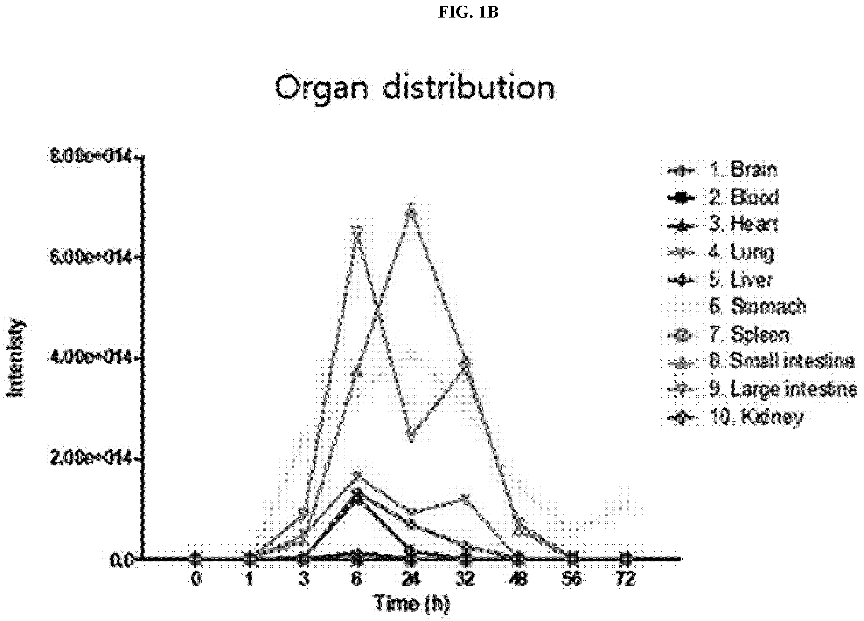

FIGS. 1A and 1B show the results of analyzing the absorption, distribution, and excretion patterns in the body by orally administering vesicles derived from the *Lactobacillus paracasei* strain, a representative *Lactobacillus* bacterium, according to one embodiment of the present invention.

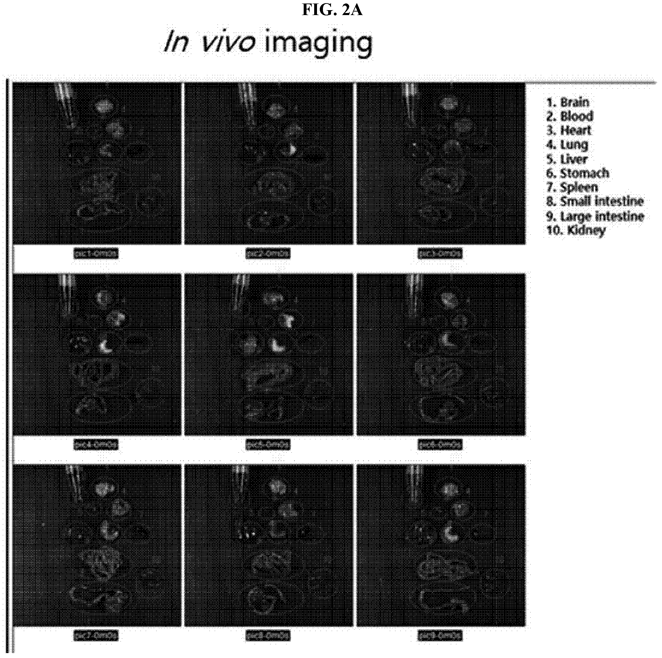

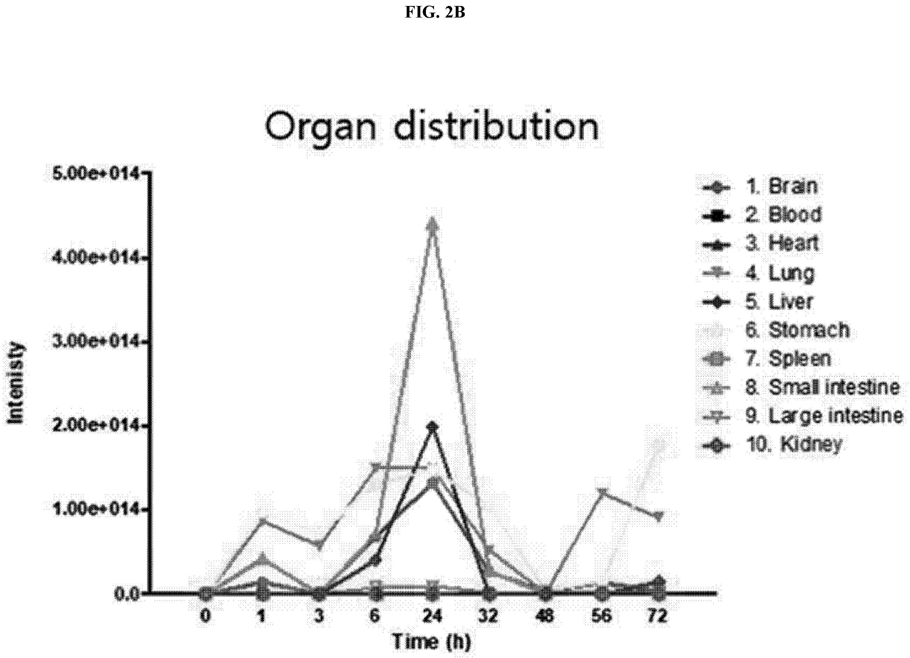

FIGS. 2A and 2B show the results of analyzing the absorption, distribution, and excretion patterns in the body by orally administering vesicles derived from the *Lactobacillus acidophilus* strain, another representative *Lactobacillus* bacterium, according to one embodiment of the present invention.

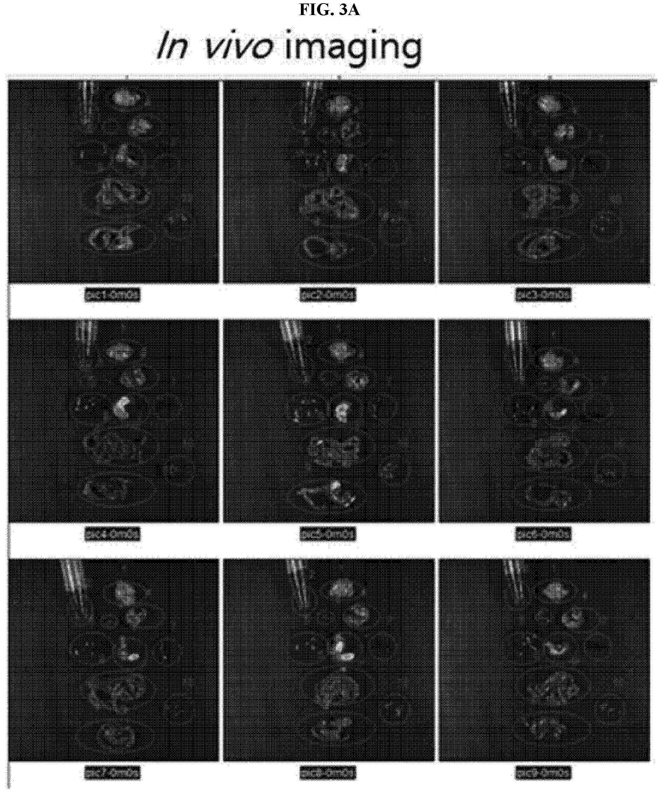

FIGS. 3A to 3C show the results of analyzing the absorption, distribution, and excretion patterns in the body by orally administering vesicles derived from the *Acinetobacter baumannii* strain, a representative Gram-negative bacterium, according to one embodiment of the present invention.

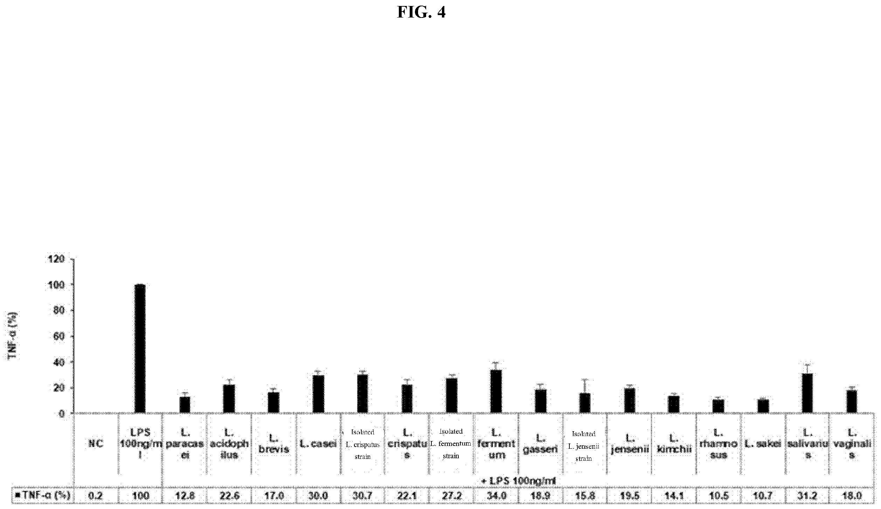

FIG. 4 shows the results of evaluating the anti-inflammatory effect of various *Lactobacillus* bacteria-derived vesicles by measuring the secretion of TNF-α, which is a representative inflammatory mediator secreted from inflammatory cells upon stimulation by lipopolysaccharide (LPS), a representative bacterial causative factor that induces inflammation, according to one embodiment of the present invention.

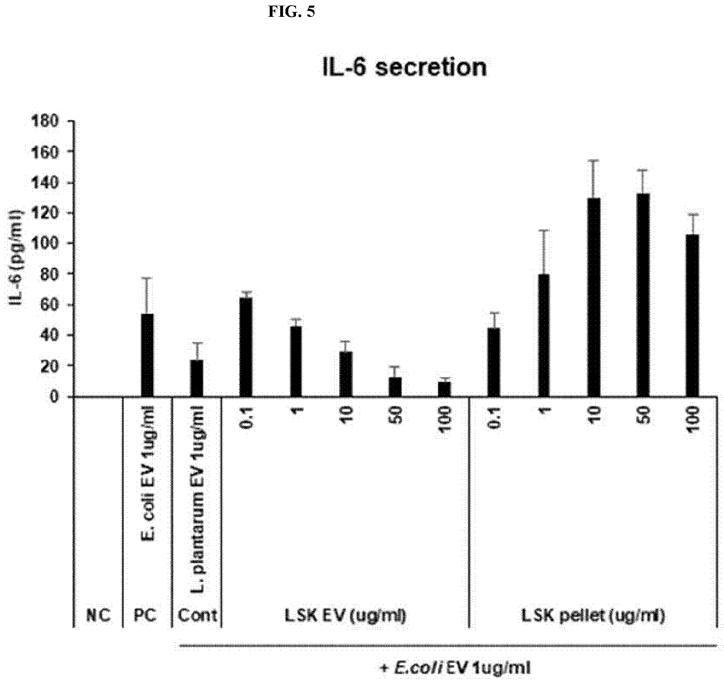

FIG. 5 shows the results of evaluating the anti-inflammatory effect of *Lactobacillus sakei* (LSK)-derived vesicles by measuring the secretion of IL-6, which is a representative inflammatory mediator secreted from inflammatory cells upon stimulation by *E. coli*-derived extracellular vesicles (*E. coli* EV), which are a representative pathogenic causative factor that induces inflammatory diseases, according to one embodiment of the present invention.

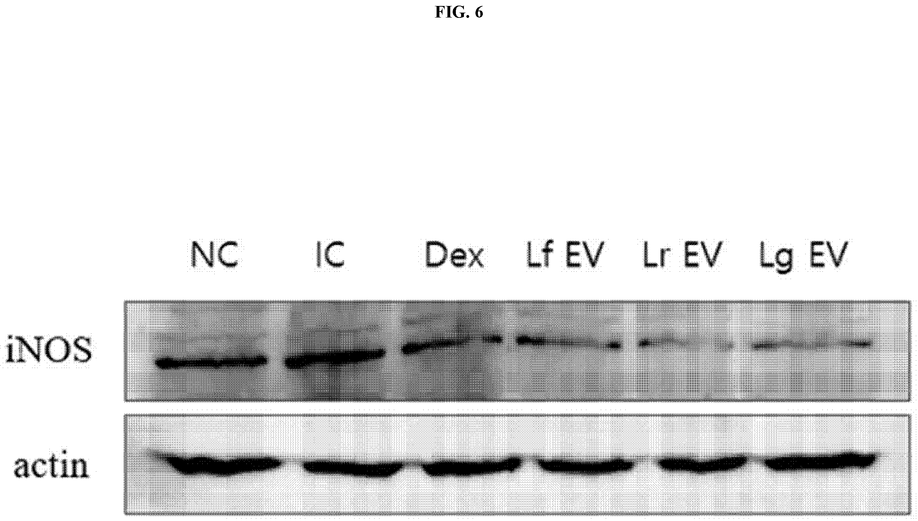

FIG. 6 shows the results of evaluating the anti-inflammatory effect of *Lactobacillus* bacteria-derived vesicles by measuring the expression of inducible NO synthase (iNOS), which causes inflammation in lung tissue, by extracting lung tissues after administering poly(I:C), a biological causative factor that simulates viral infection, into the respiratory tract, according to one embodiment of the present invention (Lf EV: *Lactobacillus fermentum-*derived vesicles; Lr EV: *L. rhamnosus-*derived vesicles; and Lg EV: *L. gasseri-derived* vesicles).

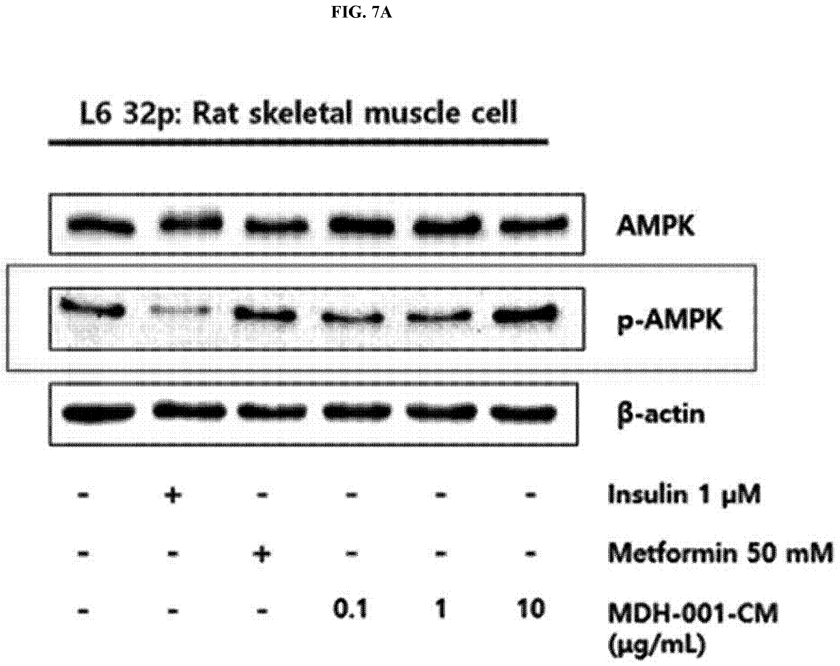

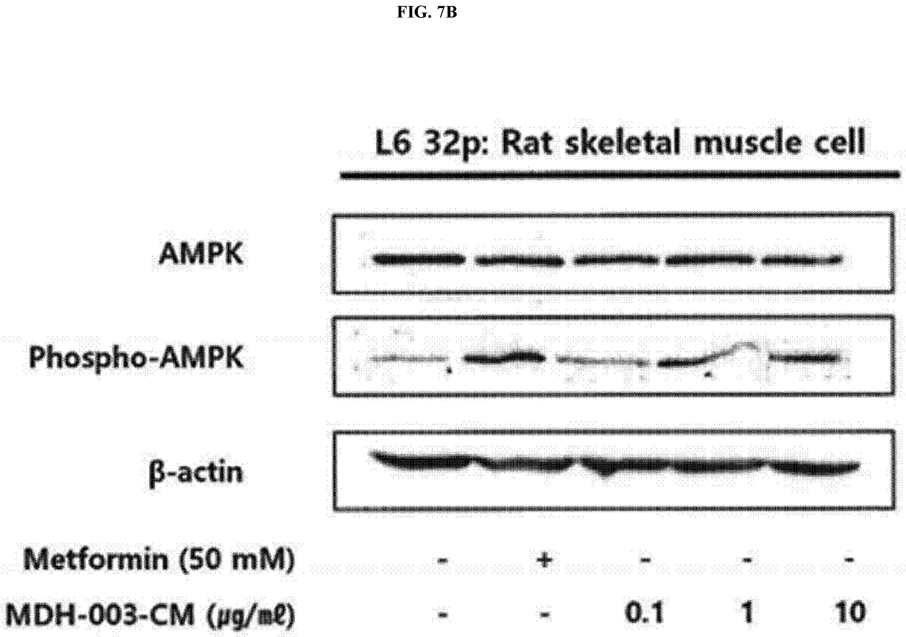

FIGS. 7A and 7B show the results of evaluating the activation of AMPK according to the treatment of myocytes with *Lactobacillus paracasei-derived* vesicles (L. *paracasei* EV) and *Lactobacillus rhamnosus-derived* vesicles (*L. rhamnosus* EV) (MDH-001-CM: *L. paracasei* EV; MDH-003-CM: *L. rhamnosus* EV). Insulin and metformin were used as controls.

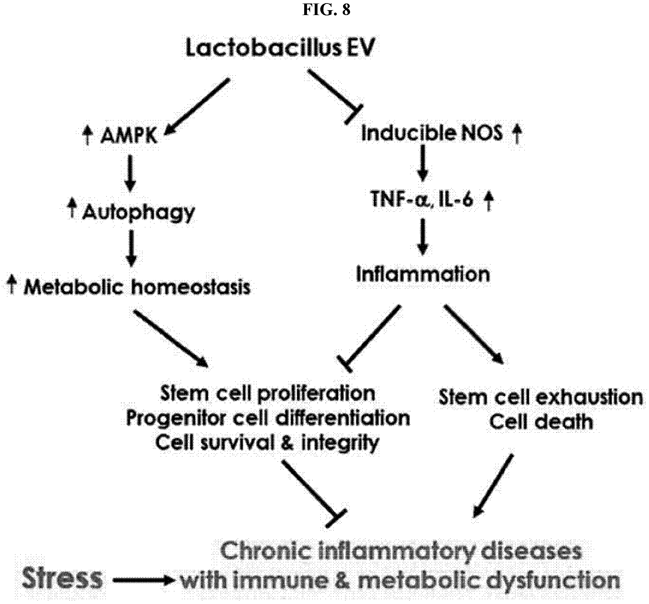

FIG. 8 is a schematic diagram of the mechanism of action of *Lactobacillus* bacteria-derived vesicles against intractable immune or metabolic dysfunction-related diseases.

### [Best Mode]

In one embodiment of the present invention, the present inventors confirmed that vesicles derived from *Lactobacillus* bacteria, which are Gram-positive bacteria, are absorbed through the stomach, small intestine, and large intestine when administered orally, and are excreted out of the body through the liver, and confirmed that they have pharmacokinetic properties that are different from Gram-negative bacteria (see Examples 2 to 4).

In another embodiment of the present invention, it was confirmed that *Lactobacillus* bacteria-derived vesicles exhibit an anti-inflammatory effect through a TNF-α inhibitory effect on inflammation caused by LPS (see Example 5).

In still another embodiment of the present invention, it was confirmed that *Lactobacillus* bacteria-derived vesicles exhibit an anti-inflammatory effect through an IL-6 inhibitory effect on inflammation caused by *E*. *coli*-derived vesicles (see Example 6).

In yet another embodiment of the present invention, it was confirmed that *Lactobacillus* bacteria-derived vesicles inhibit the expression of inducible NO synthase (iNOS) induced by poly(I:C), which is a viral causative factor (see Example 7).

In yet another embodiment of the present invention, it was confirmed that *Lactobacillus* bacteria-derived vesicles can efficiently suppress metabolic dysfunction by activating AMPK signaling (see Example 8).

Thus, the present invention is to provide a pharmaceutical composition for preventing or treating immune or metabolic dysfunction-related diseases, comprising vesicles derived from *Lactobacillus* bacteria as an active ingredient.

In the the present invention, the *Lactobacillus* bacteria may be one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae,* but are not limited thereto.

In the present invention, the "extracellular vesicle" or "vesicle" refers to a nano-sized membrane structure secreted from various bacteria. In the present invention, it collectively refers to all structures that are naturally secreted from *Lactobacillus* bacteria or artificially produced. The vesicles may be isolated by subjecting a culture medium containing *Lactobacillus* bacterial cells to one or more methods selected from the group consisting of heat treatment, centrifugation, ultrahigh-speed centrifugation, high-pressure treatment, extrusion, sonication, cell lysis, homogenization, freeze-thaw, electroporation, mechanical decomposition, chemical treatment, filtering by a filter, gel filtration chromatography, free-flow electrophoresis, and capillary electrophoresis. In addition, processes such as washing to remove impurities and concentrating the obtained vesicles may be further included.

The vesicles of the present invention may be isolated from a culture medium of *Lactobacillus* genus bacteria or from food manufactured by adding *Lactobacillus* genus bacteria, and may be naturally secreted from *Lactobacillus* genus bacteria or artificially produced, but are not limited thereto.

The method of the present invention for isolating vesicles from a culture medium or a fermented food of *Lactobacillus* genus bacteria is not particularly limited as long as it provides vesicles. For example, methods such as centrifugation, ultrahigh-speed centrifugation, filtration by a filter, gel filtration chromatography, free-flow electrophoresis, or capillary electrophoresis, and a combination thereof may be used to isolate vesicles, and processes such as washing to remove impurities and concentrating the obtained vesicles may be further included.

In the present invention, the vesicles isolated by the method described above may have an average diameter of 10-1000 nm, 10-900 nm, 10-800 nm, 10-700 nm, 10∼600 nm, 10∼500 nm, 10∼400 nm, 10∼300 nm, 10-200 nm, 10-100 nm, 10-90 nm, 10-80 nm, 10-70 nm, 10-60 nm, 10-50 nm, 10-40 nm, or 20-40 nm, but are not limited thereto.

In the present specification, "immune or metabolic dysfunction-related disease" refers to a disease caused by immune dysfunction or metabolic dysfunction in the body, and representative examples include immune diseases, metabolic diseases, malignant diseases, etc., but are not limited thereto. In addition, representative examples of the diseases include one or more selected from the group consisting of one or more skin diseases selected from the group consisting of atopic dermatitis, psoriasis, acne, and alopecia; one or more oral diseases selected from the group consisting of gingivitis and periodontitis; one or more gastrointestinal diseases selected from the group consisting of gastritis, peptic ulcers, celiac disease, food allergies, and inflammatory bowel disease; one or more hepato-biliary-pancreatic diseases selected from the group consisting of non-alcoholic steatohepatitis (NASH), alcoholic steatohepatitis, liver cirrhosis, cholangitis, cholecystitis, and pancreatitis; one or more nasal diseases selected from the group consisting of rhinitis and sinusitis; one or more pulmonary diseases selected from the group consisting of asthma, chronic obstructive pulmonary disease (COPD), pneumonia, chronic interstitial pneumonitis, and pulmonary fibrosis; one or more cardiovascular diseases selected from the group consisting of atherosclerosis, angina, metabolic syndrome, thromboembolism, myocardial infarction, cardiomyopathy, and stroke; one or more musculoskeletal diseases selected from the group consisting of rheumatoid arthritis, osteoarthritis, gout, sarcopenia, and osteoporosis; one or more neuro-psychiatric disorders selected from the group consisting of Alzheimer's disease, vascular dementia, Parkinson's disease, amyotrophic lateral sclerosis, Huntington's disease, epilepsy, multiple sclerosis, chronic inflammatory demyelinating polyneuropathy, diabetic neuropathy, autism spectrum disorder, attention deficit hyperactivity syndrome, depressive disorder, bipolar disorder, anxiety disorders, schizophrenia, obsessive compulsive disorder, post-traumatic stress disorder, dissociative disorders, eating disorders, substance use disorder, and personality disorders; and one or more ocular diseases selected from the group consisting of macular degeneration, diabetic retinopathy, glaucoma, cataracts, and dry eye, but are not limited thereto. In the present specification, "immune disease" refers to a disease caused by immune dysfunction in the body, and representative examples include one or more selected from the group consisting of one or more infectious diseases selected from the group consisting of viral infections and bacterial infections; one or more allergic diseases selected from the group consisting of atopic dermatitis, allergic rhinitis, asthma, hypersensitivity pneumonitis, food allergies, and anaphylaxis; and one or more autoimmune diseases selected from the group consisting of systemic lupus erythematosus, rheumatoid arthritis, multiple sclerosis, and Behcet's disease, but are not limited thereto.

In the present specification, "metabolic disease" refers to a disease caused by metabolic dysfunction in the body, and representative examples include diabetes, metabolic syndrome, lipid metabolism abnormalities, arteriosclerosis, and obesity, but are not limited thereto.

In the present specification, "malignant disease" refers to a disease caused by the generation of malignant cells due to metabolic dysfunction in the body, and representative examples include lung cancer, head and neck cancer, esophageal cancer, stomach cancer, colorectal cancer, colon cancer, liver cancer, bile duct cancer, pancreatic cancer, breast cancer, ovarian cancer, kidney cancer, bladder cancer, prostate cancer, thyroid cancer, brain tumors, osteosarcoma, hematologic cancer, and lymphoma, but are not limited thereto.

The amount of the vesicles in the composition of the present invention may be appropriately adjusted depending on the symptoms of a disease, the degree of progression of symptoms, the condition of a patient, and the like, and may range from, for example, 0.0001 wt% to 99.9 wt% or 0.001 wt% to 50 wt% with respect to a total weight of the composition, but the present invention is not limited thereto. The amount ratio is a value based on the amount of dried product from which a solvent is removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the patient's age, sex, and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age, and the like, the dosage is not intended to limit the scope of the present invention in any way.

In addition, the present invention provides a method for preventing or treating immune or metabolic dysfunction-related diseases, the method comprising administering a composition comprising vesicles derived from *Lactobacillus* bacteria as an active ingredient to a subject in need thereof.

In addition, the present invention provides a use of a composition comprising vesicles derived from *Lactobacillus* bacteria as an active ingredient for preventing or treating immune or metabolic dysfunction-related diseases.

In addition, the present invention provides a use of vesicles derived from *Lactobacillus* bacteria for producing drugs for treating immune or metabolic dysfunction-related diseases.

As used herein, the "subject" refers to a subject in need of treatment of a disease, and more specifically, refers to a mammal such as a human or a non-human primate, a mouse, a rat, a dog, a cat, a horse, and a cow, but the present invention is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

Meanwhile, the present invention relates to a food composition for preventing or alleviating immune or metabolic dysfunction-related diseases; or aging, comprising vesicles derived from *Lactobacillus* bacteria as an active ingredient.

The food composition may be a health functional food composition, but is not limited thereto.

The vesicles according to the present invention may be used by adding the vesicles as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

In addition, the present invention relates to provide a cosmetic composition for preventing or alleviating immune or metabolic dysfunction-related diseases; or skin aging, comprising vesicles derived from *Lactobacillus* bacteria as an active ingredient.

In the present invention, "aging" collectively refers to all physiological changes in the body that occur over time and means a life phenomenon that occurs in many different ways depending on the individual due to numerous factors. When looking at the aging phenomenon in detail, changes in the function of each component organ and tissue occur, and the aging of an individual is ultimately caused by the aging of the cells that constitute the individual. In the present invention, the aging may be due to cellular aging of the brain, liver, lungs, kidneys, muscles, skin, or of the cells of these organs, but is not limited thereto.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

The water-soluble vitamin may be any substance that is blendable with cosmetics, but examples thereof include vitamin B1, vitamin B2, vitamin B6, pyridoxine, pyridoxine hydrochloride, vitamin B 12, pantothenic acid, nicotinic acid, nicotinic acid amide, folic acid, vitamin C, vitamin H, and the like, and salts thereof (thiamine hydrochloride, sodium ascorbate, and the like) or derivatives thereof (sodium ascorbic acid-2-phosphate, magnesium ascorbic acid-2-phosphate, and the like) are also included in water-soluble vitamins that may be used in the present invention. These water-soluble vitamins may be obtained by a conventional method such as microbial transformation, purification from a microbial culture, an enzyme method, or a chemical synthesis method.

The oil-soluble vitamins may be any substance that is blendable with cosmetics, but examples thereof include vitamin A, carotene, vitamin D2, vitamin D3, vitamin E (d1-α-tocopherol, d-α-tocopherol), or the like, and derivatives thereof (e.g., ascorbyl palmitate, ascorbyl stearate, ascorbyl dipalmitate, d1-α-tocopherol acetate, d1-α-tocopherol nicotinate, vitamin E, DL-pantothenyl alcohol, D-pantothenyl alcohol, pantothenyl ethylether) may also be included in the oil-soluble vitamins used in the present invention. These oil-soluble vitamins may be obtained by a conventional method such as microbial transformation, purification from a microbial culture, or enzymatic or chemical synthesis.

The polymer peptides may be any substance that is blendable with cosmetics, but examples thereof may include collagen, hydrolyzed collagen, gelatin, elastin, hydrolyzed elastin, and keratin. The polymer peptides may be purified and obtained by any conventional method such as purification from a microbial culture, an enzyme method, or a chemical synthesis method, or may generally be used by being purified from natural substances such as the dermis of a pig, a cow, or the like and silk fiber of silkworms.

The polymeric polysaccharides may be any substance that is blendable with cosmetics, and examples thereof may include hydroxyethyl cellulose, xanthan gum, sodium hyaluronate, and chondroitin sulfate or salts thereof (sodium salts). For example, chondroitin sulfate or salts thereof may generally be purified from mammals or fish and used.

The sphingolipids may be any substance that is blendable with cosmetics, and examples thereof may include ceramide, phytosphingosine, and sphingoglycolipid. The sphingolipids may be purified, by a conventional method, from mammals, fish, shellfish, yeast, or plants, or may be obtained by a chemical synthesis method.

The cosmetic composition of the present invention may include, as necessary, other ingredients mixed in conventional cosmetics along with the above essential ingredients.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alpha-olefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/methylcetyloxysiloxane copolymers, dimethylsiloxane/methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyl-taurine.; N-acyl alkaline amino acids such as N-epsilon-lauroyl-L-lysine, N-epsilon-palmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, micro-crystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

In addition, the present invention is to provide an inhalant composition for preventing or alleviating immune or metabolic dysfunction-related diseases, comprising vesicles derived from *Lactobacillus* bacteria as an active ingredient.

An inhalation administration may be formulated according to methods known in the art and may be conveniently delivered in the form of an aerosol spray from a pressurized pack or nebulizer using a suitable propellant, such as dichlorofluoromethane, trichlorofluoromethane, dichlorotetrafluoroethane, carbon dioxide or other suitable gases. In the case of a pressurized aerosol, the dosage unit may be determined by providing a valve that delivers a metered amount. For example, gelatin capsules and cartridges for use in inhalers or insufflators may be formulated to contain a powder mixture of a compound and a suitable powder base such as lactose or starch.

In addition, the present invention relates to a composition for delivering a disease-treating drug, comprising vesicles derived from *Lactobacillus* bacteria as an active ingredient.

The term "drug delivery" used in the present invention refers to any means or act of loading and delivering a drug in the composition according to the present invention in order to deliver the drug to a specific organ, tissue, cell or organelle.

In the present invention, the composition for drug delivery may deliver a drug to one or more organs selected from the group consisting of the stomach, small intestine, large intestine, lungs, liver, kidneys, and brain, but is not limited thereto.

In addition, the present invention provides a disease-treating drug delivery system, comprising *Lactobacillus* bacteria-derived vesicles as an active ingredient.

In addition, the present invention provides a method of delivering a drug for treating disease, which comprises administering a composition comprising *Lactobacillus* bacteria-derived vesicles carrying a desired disease-treating drug as an active ingredient to a subject in need thereof.

In addition, the present invention provides a use of a composition comprising *Lactobacillus* bacteria-derived vesicles as an active ingredient for delivering a diseasetreating drug.

In addition, the present invention provides a use of *Lactobacillus* bacteria-derived vesicles for preparing a preparation for delivering a disease-treating drug.

### [Modes of the Invention]

Hereinafter, preferred Examples for helping the understanding of the present invention will be suggested. However, the following Examples are provided only to more easily understand the present invention, and the contents of the present invention are not limited by the following Examples.

### [Examples]

### Example 1: Isolation of Lactobacillus bacteria-derived vesicles

To isolate *Lactobacillus* bacteria-derived extracellular vesicles (EVs), various *Lactobacillus* bacteria were inoculated into an in-house developed medium and cultured at 37 °C at 200 rpm until the absorbance (OD_{600 nm}) became 1.0 to 1.5, and then the cells were re-inoculated into an LB (Luria Bertani) medium and cultured. Subsequently, the culture medium containing the bacterial cells was recovered and centrifuged at 10,000 g at 4 °C for 20 minutes to obtain a supernatant from which the bacterial cells were removed. The obtained supernatant was filtered again by using a 0.22 µm filter, and the filtered supernatant was concentrated to a volume of 50 mL or less using a 100 kDa Pellicon 2 Cassette filter membrane (Merck Millipore) and MasterFlex pump system (Cole-Parmer). The concentrated supernatant was filtered again by using a 0.22 µm filter to isolate *Lactobacillus* bacteria-derived vesicles. The amount of proteins contained in the supernatant was measured by using the Pierce BCA Protein Assay kit (Thermo Fisher Scientific).

### Example 2: Evaluation of pharmacokinetic properties of Lactobacillus paracasei-derived vesicles

To investigate the pharmacokinetic properties of *Lactobacillus* bacteria-derived vesicles such as absorption, distribution, and excretion upon oral administration, vesicles derived from *Lactobacillus paracasei,* a representative *Lactobacillus* bacterium, were stained with s Cy7-NHS fluorescent staining reagent and then orally administered to mice, and the fluorescence expressed in the body and each organ was measured from immediately before administration to 72 hours after administration.

As a result, as shown in FIG. 1A, it was confirmed that, when the fluorescently stained *Lactobacillus paracasei-derived* vesicles were orally administered, the vesicles passed through the gastrointestinal barrier and were systemically absorbed and distributed over time.

In addition, after orally administrating the vesicles, major organs were extracted, and the distribution pattern for each organ was observed over time.

As a result, as shown in FIG. 1B, fluorescent signals of the *Lactobacillus paracasei*-derived vesicles were observed in the stomach 1 hour after oral administration; fluorescent signals were observed in the small intestine, large intestine, and lungs 3 hours later; and fluorescent signals were also observed in the liver and brain 6 hours after oral administration. In addition, it was confirmed that most fluorescent signals disappeared in most organs 48 hours after oral administration.

From the results described above, it can be seen that when the *Lactobacillus paracasei*-derived vesicles are orally administered, they are absorbed through the stomach, small intestine, and large intestine, and after being absorbed into the blood vessels through the lymphatic vessels, they are distributed in organs such as the lungs, liver, and brain, and then excreted out of the body as excrement mostly through the liver.

### Example 3: Evaluation of pharmacokinetic properties of Lactobacillus acidophilus-derived vesicles

To evaluate whether the pharmacokinetic properties such as absorption, distribution, and excretion upon oral administration of the *Lactobacillus* bacteria-derived vesicles are common to bacteria belonging to the genus *Lactobacillus,* vesicles derived from *Lactobacillus acidophilus,* another *Lactobacillus* bacterium, were stained with a fluorescent staining reagent and then orally administered to mice, and the fluorescence expressed in the body and each organ was measured from immediately before administration to 72 hours after administration.

As a result, as shown in FIG. 2A, it was confirmed that, when the fluorescently stained *Lactobacillus acidophilus*-d*e*rived vesicles were orally administered, the vesicles passed through the gastrointestinal barrier and were systemically absorbed and distributed over time.

In addition, after orally administrating the vesicles, major organs were extracted, and the distribution pattern for each organ was observed over time.

As a result, as shown in FIG. 2B, fluorescent signals of the *Lactobacillus acidophilus-derived* vesicles were observed in the stomach, small intestine, and lungs 1 hour after oral administration; and fluorescent signals were observed in the large intestine, liver, and brain 6 hours after oral administration. In addition, it was confirmed that most fluorescent signals disappeared in most organs 48 hours after oral administration.

From the results described above, it can be seen that when the *Lactobacillus acidophilus-derived* vesicles are orally administered, they are also absorbed through the stomach, small intestine, and large intestine, and after being absorbed into the blood vessels through the lymphatic vessels, they are distributed in organs such as the lungs, liver, and brain, and then excreted out of the body as excrement mostly through the liver.

### Example 4: Evaluation of pharmacokinetic properties of Acinetobacterbacteria-derived vesicles

To evaluate whether the pharmacokinetic properties such as absorption, distribution, and excretion of the vesicles derived from Gram-positive bacteria such as *Lactobacillus* bacteria are a phenomenon specific to Gram-positive bacteria, vesicles derived from *Acinetobactor baumannii,* a representative Gram-negative bacterium, were stained with a fluorescent staining reagent and then orally administered to mice, and the expressed fluorescence was measured in the same manner as in Example 2.

As shown in FIG. 3A, it was confirmed that the strongest fluorescence signal of *Acinetobacter baumannii-derived* vesicles was observed in the stomach 3 hours after oral administration, and the fluorescence signal confirmed in the stomach decreased over time.

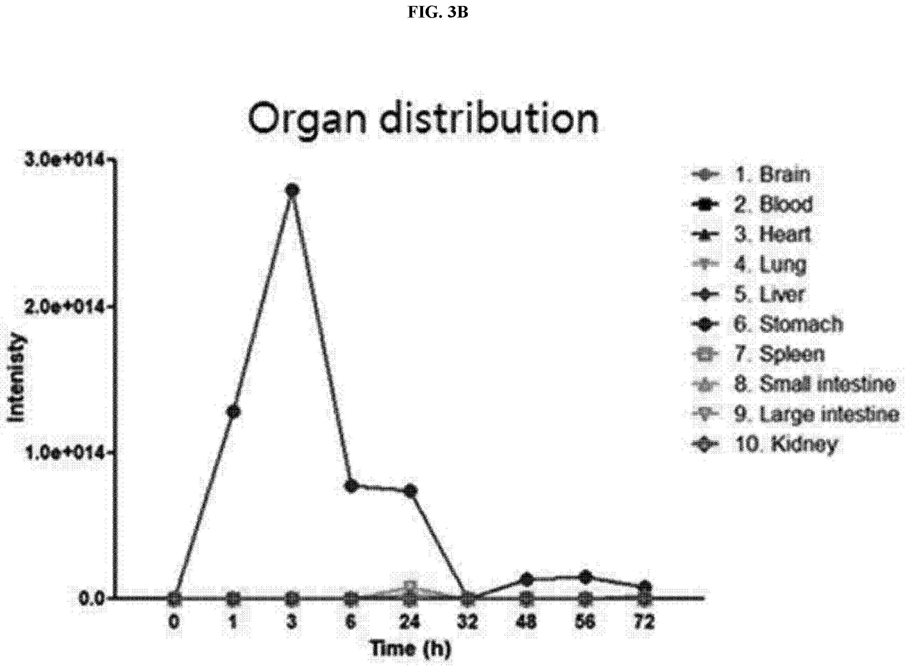

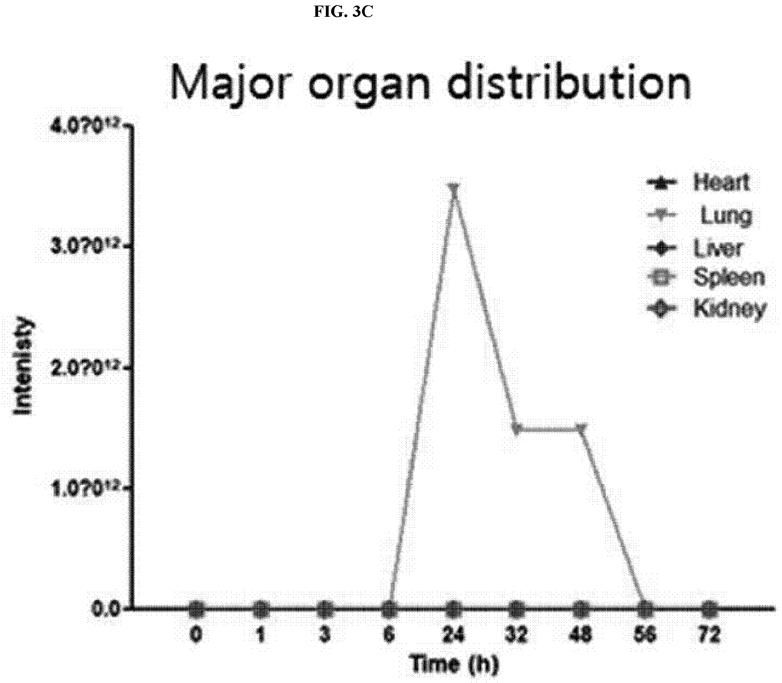

In addition, as shown in FIGS. 3B and 3C, when *Acinetobacter baumannii-*derived vesicles were orally administered, fluorescent signals were observed in stomach tissue 1 hour after administration and in lung tissue 6 hours after administration. However, unlike the pharmacokinetic properties of the *Lactobacillus* bacteria-derived vesicles in Examples 2 and 3, fluorescent signals were not measured in organs such as the liver and brain.

From the results described above, it can be seen that the pharmacokinetic properties of Gram-positive bacteria-derived vesicles and Gram-negative bacteria-derived vesicles are very different, and while Gram-positive bacteria-derived vesicles such as *Lactobacillus* bacteria-derived vesicles are distributed in organs such as the liver and brain upon oral administration and then excreted as excrement mostly through the liver, Gram-negative bacteria-derived vesicles such as *Acinetobacter* bacteria-derived vesicles are mostly absorbed through the stomach upon oral administration, distributed in the lungs, and then excreted out of the body mostly through exhalation.

### Example 5. Anti-inflammatory effect of Lactobacillus bacteria-derived vesicles on the occurrence of inflammation caused by LPS, pathogenic bacteria-derived causative factor

To confirm the anti-inflammatory effect of *Lactobacillus* bacteria-derived vesicles, Raw 264.7, a mouse macrophage cell line, was pretreated with various *Lactobacillus* bacteria-derived vesicles at a concentration of 10 µg/mL and cultured, and then the secretion amount of TNF-α was measured.

Specifically, *Lactobacillus paracasei, L. acidophilus, L. brevis, L. casei,* an in-house isolated *L. crispatus* strain, a standard *L. crispatus* strain, an in-house isolated *L. fermentum* strain, a standard *L. fermentum* strain, *L. jensenii, L. kimchii, L. rhamnosus, L. sakei, L. salivarius, L. vaginalis,* etc. were cultured by the method of Example 1 to isolate vesicles. Afterwards, Raw 264.7 cells were dispensed into a 48-well plate at 5×10⁵ cells each, treated with the *Lactobacillus* bacteria-derived vesicles diluted with a DMEM serum-free medium, and then cultured for 12 hours. Afterwards, the cells were treated with lipopolysaccharide (LPS), an inflammatory factor derived from pathogenic Gram-negative bacteria, at a concentration of 100 ng/mL and then further cultured for 12 hours. Afterwards, the secretion amount of TNF-α was measured by using an ELISA method.

For the ELISA method, a capture antibody (Abcam) was diluted in a phosphate buffer solution, and the resulting mixture was dispensed into a 96-well plate at 50 µL each, and allowed to react at 4 °C for 16 hours. In addition, after washing the product three times using 100 µL of a phosphate buffer solution (PBST) containing 0.05% Tween-20, 100 µL of a phosphate buffer solution (RD) containing 1% bovine serum albumin (BSA) was added to each well for blocking at room temperature for 1 hour. Then, 50 µL of each of the samples and the standard to be tested were added at the same concentration and allowed to react at room temperature for 2 hours, and then the product was washed three times using PBST to completely remove unbound antibodies. Afterwards, streptavidin-HRP (R&D Systems) diluted 1/40 in RD was added at a volume of 50 µL per well and allowed to react at room temperature for 20 minutes. Finally, after washing the product three times with 100 µL of PBST, 50 µL of TMB substrate (SurModics) was added per well. When color development progressed for 5 to 20 minutes, 50 µL of a 1 M sulfuric acid solution was added per well to terminate the reaction, and then the absorbance was measured at 450 nm by using a SpectraMax M3 microplate reader (Molecular Devices).

As a result, as shown in FIG. 4, it was confirmed that, when *Lactobacillus* bacteria-derived vesicles were administered, the secretion of TNF-α induced by LPS in macrophages was reduced by 50% or more, regardless of the treated *Lactobacillus* strain. Through the results described above, it was confirmed that *Lactobacillus* bacteria-derived vesicles can effectively suppress inflammation caused by LPS derived from pathogenic bacteria.

### Example 6. Anti-inflammatory effect of Lactobacillus bacteria-derived vesicles on the occurrence of inflammation caused by E. coli-derived vesicles, which are pathogenic bacteria-derived vesicles

To compare the anti-inflammatory effects of *Lactobacillus* bacteria and *Lactobacillus-derived* vesicles, Raw 264.7, a mouse macrophage cell line, was treated with the *Lactobacillus sakei* (LSK) strain and *Lactobacillus sakei-derived* vesicles at concentrations of 0.1, 1, 10, 50, and 100 µg/mL, and the effect of *E. coli*-derived vesicles (*E. coli* EV), a pathogenic factor, on the secretion of IL-6 was evaluated. More specifically, Raw 264.7 cells were dispensed into a 48-well plate at 5×10⁵ cells each and the macrophage cells were treated with the *Lactobacillus sakei* strain (pellet) or *Lactobacillus sakei-derived* vesicles diluted with a DMEM serum-free medium and then cultured for 12 hours. Then, after treating the cells with *E. coli*-derived vesicles at a concentration of 1 µg/mL and further culturing for 12 hours, the concentration of IL-6 was measured by an ELISA method. As a control drug, vesicles from *Lactobacillus plantarum,* another *Lactobacillus* bacterium, were pretreated at a concentration of 1 µg/mL.

As a result, as shown in FIG. 5, when *Lactobacillus* plantarum and *Lactobacillus* sakei-derived vesicles were pretreated, IL-6 secretion due to the *E. coli-*derived vesicles was suppressed, and in the case of *Lactobacillus sakei-derived* vesicles, IL-6 secretion was inhibited in a dose-dependent manner. On the other hand, when the *Lactobacillus sakei* strain was administered, IL-6 secretion due to *E. coli-*derived vesicles rather increased. Through the results described above, it was confirmed that *Lactobacillus* bacteria cannot suppress the inflammation caused by vesicles secreted by pathogenic bacteria, but *Lactobacillus* bacteria-derived vesicles can efficiently suppress the inflammation.

### Example 7. Inhibitory effect of Lactobacillus bacteria-derived vesicles on inducible NO synthase (iNOS) expression induced by poly(I:C), viral causative factor

When cells are repeatedly exposed to various stresses, cellular aging occurs due to oxidative stress, which causes cellular dysfunction, and cell death is also caused, resulting in cellular aging-related diseases. In particular, high-concentrations of NO, produced through iNOS signaling, causes excessive stress in cells, resulting in immune dysfunction and metabolic dysfunction and thereby promoting the development of diseases. Meanwhile, poly(I:C), which is an artificially synthesized doublestructured ribonucleotide polymer, is structurally similar to dsRNA that is present in some viruses and activates various inflammatory signaling pathways, thereby causing viral infectious diseases.

To evaluate the therapeutic effect of *Lactobacillus* bacteria-derived vesicles on the development of cellular aging-related diseases caused by immune dysfunction caused by viral causative agents, an immune disease mouse model was created by administering poly(I:C), a viral causative agent, through the respiratory tract to C57BL/6 6-week-old male mice every other day for two weeks. Afterwards, lung tissue was extracted and the iNOS expression pattern was evaluated by the Western blotting method. The therapeutic effect of *Lactobacillus* bacteria-derived vesicles was evaluated by orally administering 50 µg of *Lactobacillus fermentum-*derived vesicles (Lf EV), 50 µg of *Lactobacillus rhamnosus-derived* vesicles (Lr EV), and 50 µg of *Lactobacillus gasseri-*derived vesicles (Lg EV), and as a control drug, 1 mg of dexamethasone (Dex) was intraperitoneally administered.

As a result, as shown in FIG. 6, the expression of iNOS due to poly(I:C) was efficiently inhibited in the *Lactobacillus fermentum-*derived vesicles (Lf EV), *Lactobacillus rhamnosus-derived* vesicles (Lr EV), and *Lactobacillus gasseri*-derived vesicles (Lg EV), and the inhibitory effect was better than that of dexamethasone, which was the positive control drug.

From the results described above, it can be seen that *Lactobacillus* bacteria-derived vesicles can efficiently suppress immune dysfunction caused by viral causative factors and efficiently treat cellular aging- and inflammation-related diseases.

### Example 8. Effect of Lactobacillus bacteria-derived vesicles on the activation of AMPK signaling in myocytes

During fasting or exercising, myocytes are depleted of energy, which causes a decrease in ATP production and an increase in AMP production. It is well known that, in this case, cells activate AMPK signaling to increase cellular homeostasis against metabolic stress. To evaluate a metabolic function regulating effect of *Lactobacillus* bacteria-derived vesicles, myocytes were each treated with *Lactobacillus paracasei*-derived vesicles (MDH-001-CM) and *Lactobacillus rhamnosus*-derived vesicles (MDH-003-CM) at concentrations of 0, 0.1, 1, and 10 µg/ml for 1 hour. Afterwards, the degree of AMPK phosphorylation (pAMPK), an indicator of AMPK signaling, was measured by Western blotting. As control drugs, 1 µM insulin and 50 mM metformin were used.

As a result, as shown in FIG. 7A, the phosphorylation of AMPK (pAMPK) was inhibited by the control drug insulin and but increased by metformin. In addition, when *Lactobacillus paracasei-derived* vesicles were treated, the expression of pAMPK increased in a vesicle concentration-dependent manner.

In addition, as shown in FIG. 7B, when *Lactobacillus rhamnosus-derived* vesicles were treated, the expression of pAMPK increased in a vesicle concentration-dependent manner. Through the results described above, it can be seen that *Lactobacillus* bacteria-derived vesicles can efficiently suppress metabolic dysfunction by activating AMPK signaling.

Since it was confirmed through the results described above that the *Lactobacillus* bacteria-derived vesicles of the present invention can efficiently treat diseases by regulating immune dysfunction to suppress inflammation and by regulating metabolic dysfunction to increase cellular homeostasis (see FIG. 8), the *Lactobacillus* bacteria-derived vesicles of the present invention are expected to be used for use in alleviating, preventing or treating diseases caused by immune dysfunction or metabolic dysfunction.

The above-described description of the present invention is merely provided to exemplify the present invention, and it will be understood by those of ordinary skill in the art to which the present invention belongs that the present invention can be implemented in modified forms without departing from the essential features of the present invention. Therefore, the exemplary embodiments described above should be interpreted as illustrative and not limited in any aspect.

### [Industrial Applicability]

The *Lactobacillus* bacteria-derived vesicles according to the present invention are capable of not only regulating inflammatory responses by suppressing NF-κB signaling, a key signal of inflammatory responses but also activating AMPK signaling in metabolic stress situations where ATP production is poor, thereby increasing cellular homeostasis. Therefore, the vesicles may be effectively used in the development of pharmaceuticals or health functional foods to alleviate, prevent, or treat diseases caused by immune dysfunction or metabolic dysfunction, and thus have industrial applicability.

## Claims

1. A pharmaceutical composition for preventing or treating immune or metabolic dysfunction-related diseases, comprising vesicles derived from *Lactobacillus* bacteria as an active ingredient.

2. The pharmaceutical composition of claim 1, wherein the immune or metabolic dysfunction-related diseases is one or more selected from the group consisting of immune diseases, metabolic diseases, and malignant diseases.

3. The pharmaceutical composition of claim 1, wherein the immune or metabolic dysfunction-related diseases is one or more selected from the group consisting of the following diseases:
one or more skin diseases selected from the group consisting of atopic dermatitis, psoriasis, acne, and alopecia;
one or more oral diseases selected from the group consisting of gingivitis and periodontitis;
one or more gastrointestinal diseases selected from the group consisting of gastritis, peptic ulcers, celiac disease, food allergies, and inflammatory bowel disease;
one or more hepato-biliary-pancreatic diseases selected from the group consisting of non-alcoholic steatohepatitis (NASH), alcoholic steatohepatitis, liver cirrhosis, cholangitis, cholecystitis, and pancreatitis;
one or more nasal diseases selected from the group consisting of rhinitis and sinusitis;
one or more pulmonary diseases selected from the group consisting of asthma, chronic obstructive pulmonary disease (COPD), pneumonia, chronic interstitial pneumonitis, and pulmonary fibrosis;
one or more cardiovascular diseases selected from the group consisting of atherosclerosis, angina, metabolic syndrome, thromboembolism, myocardial infarction, cardiomyopathy, and stroke;
one or more musculoskeletal diseases selected from the group consisting of rheumatoid arthritis, osteoarthritis, gout, sarcopenia, and osteoporosis;
one or more neuro-psychiatric disorders selected from the group consisting of Alzheimer's disease, vascular dementia, Parkinson's disease, amyotrophic lateral sclerosis, Huntington's disease, epilepsy, multiple sclerosis, chronic inflammatory demyelinating polyneuropathy, diabetic neuropathy, autism spectrum disorder, attention deficit hyperactivity syndrome, depressive disorder, bipolar disorder, anxiety disorders, schizophrenia, obsessive compulsive disorder, post-traumatic stress disorder, dissociative disorders, eating disorders, substance use disorder, and personality disorders; and
one or more ocular diseases selected from the group consisting of macular degeneration, diabetic retinopathy, glaucoma, cataracts, and dry eye.

4. The pharmaceutical composition of claim 2, wherein the immune diseases is one or more selected from the group consisting of the following diseases:
one or more infectious diseases selected from the group consisting of viral infections and bacterial infections;
one or more allergic diseases selected from the group consisting of atopic dermatitis, allergic rhinitis, asthma, hypersensitivity pneumonitis, food allergies, and anaphylaxis; and
one or more autoimmune diseases selected from the group consisting of systemic lupus erythematosus, rheumatoid arthritis, multiple sclerosis, and Behcet's disease.

5. The pharmaceutical composition of claim 2, wherein the metabolic disease is one or more selected from the group consisting of diabetes, metabolic syndrome, lipid metabolism abnormalities, arteriosclerosis, and obesity.

6. The pharmaceutical composition of claim 2, wherein the malignant disease is one or more selected from the group consisting of lung cancer, head and neck cancer, esophageal cancer, stomach cancer, colorectal cancer, colon cancer, liver cancer, bile duct cancer, pancreatic cancer, breast cancer, ovarian cancer, kidney cancer, bladder cancer, prostate cancer, thyroid cancer, brain tumors, osteosarcoma, hematologic cancer, and lymphoma.

7. The pharmaceutical composition of claim 1, wherein the immune or metabolic dysfunction-related disease is a disease mediated by inducible NO synthase (iNOS).

8. The pharmaceutical composition of claim 1, wherein the immune or metabolic dysfunction-related disease is a disease mediated by TNF-α or IL-6

9. The pharmaceutical composition of claim 1, wherein the *Lactobacillus* bacteria is one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae.*

10. The pharmaceutical composition of claim 1, wherein the vesicles have an average diameter of 10 to 300 nm.

11. The pharmaceutical composition of claim 1, wherein the vesicles are naturally secreted or artificially produced from *Lactobacillus* bacteria.

12. The pharmaceutical composition of claim 1, the vesicles are isolated from a culture medium of *Lactobacillus* bacteria or food cultured by adding *Lactobacillus* bacteria.

13. A food composition for preventing or alleviating immune or metabolic dysfunction-related diseases; or aging, comprising vesicles derived from *Lactobacillus* bacteria as an active ingredient.

14. The food composition of claim 13, wherein the immune or metabolic dysfunction-related diseases is one or more selected from the group consisting of immune diseases, metabolic diseases, and malignant diseases.

15. The food composition of claim 13, wherein the immune or metabolic dysfunction-related diseases is one or more selected from the group consisting of the following diseases:
one or more skin diseases selected from the group consisting of atopic dermatitis, psoriasis, acne, and alopecia;
one or more oral diseases selected from the group consisting of gingivitis and periodontitis;
one or more gastrointestinal diseases selected from the group consisting of gastritis, peptic ulcers, celiac disease, food allergies, and inflammatory bowel disease;
one or more hepato-biliary-pancreatic diseases selected from the group consisting of non-alcoholic steatohepatitis (NASH), alcoholic steatohepatitis, liver cirrhosis, cholangitis, cholecystitis, and pancreatitis;
one or more nasal diseases selected from the group consisting of rhinitis and sinusitis;
one or more pulmonary diseases selected from the group consisting of asthma, chronic obstructive pulmonary disease (COPD), pneumonia, chronic interstitial pneumonitis, and pulmonary fibrosis;
one or more cardiovascular diseases selected from the group consisting of atherosclerosis, angina, metabolic syndrome, thromboembolism, myocardial infarction, cardiomyopathy, and stroke;
one or more musculoskeletal diseases selected from the group consisting of rheumatoid arthritis, osteoarthritis, gout, sarcopenia, and osteoporosis;
one or more neuro-psychiatric disorders selected from the group consisting of Alzheimer's disease, vascular dementia, Parkinson's disease, amyotrophic lateral sclerosis, Huntington's disease, epilepsy, multiple sclerosis, chronic inflammatory demyelinating polyneuropathy, diabetic neuropathy, autism spectrum disorder, attention deficit hyperactivity syndrome, depressive disorder, bipolar disorder, anxiety disorders, schizophrenia, obsessive compulsive disorder, post-traumatic stress disorder, dissociative disorders, eating disorders, substance use disorder, and personality disorders; and
one or more ocular diseases selected from the group consisting of macular degeneration, diabetic retinopathy, glaucoma, cataracts, and dry eye.

16. The food composition of claim 14, wherein the immune diseases is one or more selected from the group consisting of the following diseases:
one or more infectious diseases selected from the group consisting of viral infections and bacterial infections;
one or more allergic diseases selected from the group consisting of atopic dermatitis, allergic rhinitis, asthma, hypersensitivity pneumonitis, food allergies, and anaphylaxis; and
one or more autoimmune diseases selected from the group consisting of systemic lupus erythematosus, rheumatoid arthritis, multiple sclerosis, and Behcet's disease.

17. The food composition of claim 14, wherein the metabolic disease is one or more selected from the group consisting of diabetes, metabolic syndrome, lipid metabolism abnormalities, arteriosclerosis, and obesity.

18. The food composition of claim 14, wherein the malignant disease is one or more selected from the group consisting of lung cancer, head and neck cancer, esophageal cancer, stomach cancer, colorectal cancer, colon cancer, liver cancer, bile duct cancer, pancreatic cancer, breast cancer, ovarian cancer, kidney cancer, bladder cancer, prostate cancer, thyroid cancer, brain tumors, osteosarcoma, hematologic cancer, and lymphoma.

19. The food composition of claim 13, wherein the *Lactobacillus* bacteria is one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae.*

20. The food composition of claim 13, the vesicles are isolated from a culture medium of *Lactobacillus* bacteria or food cultured by adding *Lactobacillus* bacteria.

21. An inhalant composition for preventing or alleviating immune or metabolic dysfunction-related diseases, comprising vesicles derived from *Lactobacillus* bacteria as an active ingredient.

22. A cosmetic composition for preventing or alleviating immune or metabolic dysfunction-related diseases; or skin aging, comprising vesicles derived from *Lactobacillus* bacteria as an active ingredient.

23. The cosmetic composition of claim 22, wherein the immune or metabolic dysfunction-related diseases is one or more skin diseases selected from the group consisting of atopic dermatitis, psoriasis, acne, and alopecia.

24. The cosmetic composition of claim 22, wherein the *Lactobacillus* bacteria is one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae.*

25. A composition for delivering a disease-treating drug, comprising vesicles derived from *Lactobacillus* bacteria as an active ingredient.

26. The composition of claim 25, wherein the disease is one or more selected from the group consisting of the following diseases:
one or more skin diseases selected from the group consisting of atopic dermatitis, psoriasis, acne, and alopecia;
one or more oral diseases selected from the group consisting of gingivitis and periodontitis;
one or more gastrointestinal diseases selected from the group consisting of gastritis, peptic ulcers, celiac disease, food allergies, and inflammatory bowel disease;
one or more hepato-biliary-pancreatic diseases selected from the group consisting of non-alcoholic steatohepatitis (NASH), alcoholic steatohepatitis, liver cirrhosis, cholangitis, cholecystitis, and pancreatitis;
one or more nasal diseases selected from the group consisting of rhinitis and sinusitis;
one or more pulmonary diseases selected from the group consisting of asthma, chronic obstructive pulmonary disease (COPD), pneumonia, chronic interstitial pneumonitis, and pulmonary fibrosis;
one or more cardiovascular diseases selected from the group consisting of atherosclerosis, angina, metabolic syndrome, thromboembolism, myocardial infarction, cardiomyopathy, and stroke;
one or more musculoskeletal diseases selected from the group consisting of rheumatoid arthritis, osteoarthritis, gout, sarcopenia, and osteoporosis;
one or more neuro-psychiatric disorders selected from the group consisting of Alzheimer's disease, vascular dementia, Parkinson's disease, amyotrophic lateral sclerosis, Huntington's disease, epilepsy, multiple sclerosis, chronic inflammatory demyelinating polyneuropathy, diabetic neuropathy, autism spectrum disorder, attention deficit hyperactivity syndrome, depressive disorder, bipolar disorder, anxiety disorders, schizophrenia, obsessive compulsive disorder, post-traumatic stress disorder, dissociative disorders, eating disorders, substance use disorder, and personality disorders; and
one or more ocular diseases selected from the group consisting of macular degeneration, diabetic retinopathy, glaucoma, cataracts, and dry eye.

27. The composition of claim 25, wherein the disease is one or more selected from the group consisting of lung cancer, head and neck cancer, esophageal cancer, stomach cancer, colorectal cancer, colon cancer, liver cancer, bile duct cancer, pancreatic cancer, breast cancer, ovarian cancer, kidney cancer, bladder cancer, prostate cancer, thyroid cancer, brain tumors, osteosarcoma, hematologic cancer, and lymphoma.

28. The composition of claim 25, wherein the *Lactobacillus* bacteria is one or more selected from the group consisting of *Lactobacillus brevis, Lactobacillus acetotolerans, Lactobacillus acidifarinae, Lactobacillus acidipiscis, Lactobacillus agilis, Lactobacillus algidus, Lactobacillus alimentarus, Lactobacillus amylolyticus, Lactobacillus amylotrophicus, Lactobacillus amylovorus, Lactobacillus animalis, Lactobacillus antri, Lactobacillus apodemi, Lactobacillus aviarius, Lactobacillus bifermentans, Lactobacillus bombicola, Lactobacillus buchneri, Lactobacillus camelliae, Lactobacillus casei, Lactobacillus catenaformis, Lactobacillus ceti, Lactobacillus coleohominis, Lactobacillus collinoides, Lactobacillus composti, Lactobacillus concavus, Lactobacillus coryniformis, Lactobacillus crispatus, Lactobacillus crustorum, Lactobacillus curvatus, Lactobacillus delbruecki, Lactobacillus dextrinicus, Lactobacillus diolivorans, Lactobacillus equi, Lactobacillus equigenerosi, Lactobacillus farraginis, Laobacillus farciminis, Lactobacillus fermentum, Lactobacillus fornicalis, Lactobacillus fructivorans, Lactobacillus frumenti, Lactobacillus fuchuensis, Lactobacillus gallinarum, Lactobacillus gasseri, Lactobacillus gastricus, Lactobacillus ghanensis, Lactobacillus graminis, Lactobacillus hammesii, Lactobacillus hamsteri, Lactobacillus harbinensis, Lactobacillus hayakitensis, Lactobacillus helveticus, Lactobacillus hilgardii, Lactobacillus homohiochii, Lactobacillus iners, Lactobacillus ingluviei, Lactobacillus intestinalis, Lactobacillus jensenii, Lactobacillus johnsonii, Lactobacillus kalixensis, Lactobacillus kefiranofaciens, Lactobacillus kefiri, Lactobacillus kimchii, Lactobacillus kitasatonis, Lactobacillus kunkeei, Lactobacillus leichmannii, Lactobacillus lindneri, Lactobacillus malefermentans, Lactobacillus mali, Lactobacillus manihotivorans, Lactobacillus mindensis, Lactobacillus mucosae, Lactobacillus murinus, Lactobacillus nagelii, Lactobacillus namurensis, Lactobacillus nantensis, Lactobacillus oligofermentans, Lactobacillus oris, Lactobacillus panis, Lactobacillus pantheris, Lactobacillus parabrevis, Lactobacillus parabuchneri, Lactobacillus paracollinoides, Lactobacillus parafarraginis, Lactobacillus parakefiri, Lactobacillus paralimentarius, Lactobacillus paraplantarum, Lactobacillus pentosus, Lactobacillus perolens, Lactobacillus pontis, Lactobacillus protectus, Lactobacillus psittaci, Lactobacillus rennini, Lactobacillus reuteri, Lactobacillus rimae, Lactobacillus rogosae, Lactobacillus rossiae, Lactobacillus ruminis, Lactobacillus saerimneri, Lactobacillus sakei, Lactobacillus salivarius, Lactobacillus sanfranciscensis, Lactobacillus satsumensis, Lactobacillus secaliphilus, Lactobacillus sharpeae, Lactobacillus siliginis, Lactobacillus spicheri, Lactobacillus suebicus, Lactobacillus thailandensis, Lactobacillus ultunensis, Lactobacillus vaccinostercus, Lactobacillus vaginalis, Lactobacillus versmoldensis, Lactobacillus vini, Lactobacillus vitulinus, Lactobacillus zeae,* and *Lactobacillus zymae.*

29. A method for preventing or treating immune or metabolic dysfunction-related diseases, the method comprising administering a composition comprising vesicles derived from *Lactobacillus* bacteria as an active ingredient to a subject in need thereof.

30. A use of a composition comprising vesicles derived from *Lactobacillus* bacteria as an active ingredient for preventing or treating immune or metabolic dysfunction-related diseases.

31. A use of vesicles derived from *Lactobacillus* bacteria for producing drugs for treating immune or metabolic dysfunction-related diseases.

32. A disease-treating drug delivery system, comprising *Lactobacillus* bacteria-derived vesicles as an active ingredient.

33. A method of delivering a drug for treating disease, which comprises administering a composition comprising *Lactobacillus* bacteria-derived vesicles carrying a desired disease-treating drug as an active ingredient to a subject in need thereof.

34. A use of a composition comprising *Lactobacillus* bacteria-derived vesicles as an active ingredient for delivering a disease-treating drug.

35. A use of *Lactobacillus* bacteria-derived vesicles for preparing a preparation for delivering a disease-treating drug.
